(19) Europäisches Patentamt / European Patent Office / Office européen des brevets

(11) **EP 2 853 290 A1**

(12) **EUROPEAN PATENT APPLICATION**
published in accordance with Art. 153(4) EPC

(43) Date of publication:
01.04.2015 Bulletin 2015/14

(51) Int Cl.:
*A61N 1/39* (2006.01)  *A61N 1/04* (2006.01)
*A61B 5/0408* (2006.01)

(21) Application number: 13791036.0

(22) Date of filing: 26.04.2013

(86) International application number:
PCT/KR2013/003618

(87) International publication number:
WO 2013/172569 (21.11.2013 Gazette 2013/47)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 15.05.2012 KR 20120051535

(71) Applicant: Cu Medical Systems Inc.
Wonju-si, Gangwon-do 220-801 (KR)

(72) Inventor: CHOI, Sung Hwan
Wonju-si
Gangwon-do 220-735 (KR)

(74) Representative: Caspary, Karsten et al
Kroher-Strobel
Rechts- und Patentanwälte PartmbB
Bavariaring 20
80336 München (DE)

(54) **CARDIOVERTER INCLUDING FUNCTIONS FOR MEASURING AND REMOVING MOTION ARTIFACT**

(57)     A defibrillator including the function for measuring a motion artifact generated when pressing the heart, and a method of operating the defibrillator are provided. The defibrillator includes: a first pair of electrodes that includes a first main electrode and a first sub-electrode arranged adjacent to and insulated from the first main electrode; a second pair of electrodes that includes a second main electrode and a second sub electrode arranged adjacent to and insulated from the second main electrode; a first measuring unit that measures a first action potential associated with the first pair of electrodes by using the potential difference between the first pair of electrodes; a second measuring unit that measures a second action potential associated with the second pair of electrodes by using the potential difference between the second pair of electrodes; and an estimating unit that estimates ECG artifacts associated with the difference between the first action potential and the second action potential.

FIG. 2

EP 2 853 290 A1

**Description**

[Technical Field]

The present invention relates to a defibrillator and, more particularly, to a defibrillator having a function of measuring and removing motion artifacts.

[Background Art]

Motion artifact, in ECG sensing applications, is a signal distortion caused by motion of an ECG sensing electrode. The motion artifact is generated because a charge distribution on a boundary surface between an electrode and an electrolyte is disturbed due to the motion of the electrode, a half-cell potential is changed, and thus a change of a measured bio potential is caused. It is difficult to remove such a motion artifact when an electrocardiogram (ECG) is measured because the motion artifact chiefly has a low frequency component.

If an emergency patient occurs, a motion artifact is generated as a motion, such as the transfer of the patient, is generated. Accordingly, there is a problem in that it is difficult to check and analyze the rhythm of the heart.

Furthermore, in order to improve the restoration of spontaneous circulation of a sudden cardiac arrest patient, a chest compression hands-off time needs to be minimized. In the case of a current automated external defibrillator, cardiopulmonary resuscitation has to be stopped during a period in which the rhythm of the heart is checked for precise ECG analysis. Accordingly, there is a need for a technology for removing an artifact generated when the chest is compressed in order to continue cardiopulmonary resuscitation when an ECG is analyzed.

[Disclosure]

[Technical Problem]

An object of the present invention is to provide a defibrillator having a function of measuring and removing a motion artifact generated when the chest is compressed and a method of operating the defibrillator.

Another object of the present invention is to provide a defibrillator capable of minimizing chest compression hands-off time and increasing the accuracy of ECG measurement by measuring and removing a motion artifact generated due to a motion of a defibrillator electrode which is attributable to various causes when an ECG is measured, for example, chest compression or a motion of the patient's body during cardiopulmonary resuscitation (CPR), and a method of operating the defibrillator.

[Technical Solution]

In accordance with an aspect of the present invention, a defibrillator is provided for monitoring the ECG signal of an object using a plurality of electrodes, including a pair of first electrodes comprising a first main electrode and a first sub-electrode placed adjacent to the first main electrode and insulated from the first main electrode, a pair of second electrodes comprising a second main electrode and a second sub-electrode placed adjacent to the second main electrode and insulated from the second main electrode, a first measurement unit measuring a first active potential, associated with the pair of first electrodes, using a potential difference between the pair of first electrodes, a second measurement unit measuring a second active potential, associated with the pair of second electrodes, using a potential difference between the pair of second electrodes, and an estimation unit estimating an ECG artifact associated with a difference between the first active potential and the second active potential.

In accordance with an embodiment of the present invention, the defibrillator may further include a third measurement unit measuring a low ECG signal using a potential difference between the first main electrode and the second main electrode and a signal restoration unit restoring the final ECG obtained by removing the ECG artifact from the low ECG signal using the estimated ECG artifact.

Furthermore, the defibrillator may further include an energy conversion unit receiving an electromagnetic signal around the defibrillator and converting the electromagnetic signal into electric energy and a power storage unit storing the electric energy converted by the energy conversion unit and supplying the driving power for the defibrillator.

Furthermore, the electrode unit of a defibrillator brought in contact with an object in order to measure the ECG signal of the object is configured to include a pair of first electrodes including a first main electrode and a first sub-electrode placed adjacent to the first main electrode and insulated from the first main electrode and a pair of second electrodes including a second main electrode and a second sub-electrode placed adjacent to the second main electrode and insulated from the second main electrode.

In accordance with another aspect of the present invention, there is provided a method of operating a defibrillator for monitoring the ECG signal of an object using a plurality of electrodes, including steps of measuring a first active potential associated with a pair of first electrodes using a potential difference between the pair of first electrodes of the defibrillator - the pair of first electrodes including a first main electrode and a first sub-electrode placed adjacent to the first main electrode and insulated from the first main electrode -, measuring a second active potential associated with a pair of second electrodes using a potential difference between the pair of second electrodes of the defibrillator - the pair of second electrodes including a second main electrode and a second sub-electrode placed adjacent to the second main electrode and insulated from the second main electrode -, and estimating an ECG artifact associated with a difference between the first active potential and the second active potential.

The method of operating a defibrillator may further include steps of measuring a low ECG signal using a potential difference between the first main electrode and the second main electrode and restoring the final ECG obtained by removing an artifact from the low ECG signal using the estimated ECG artifact.

[Advantageous Effects]

In accordance with an embodiment of the present invention, a chest compression interruption time can be minimized and an ECG can be precisely measured by measuring and removing the external force induced electrical excitation of the heart or a change of chest impedance attributable to chest compression and a variety of types of noise present within an ECG, such as an artifact generated on a boundary surface between an electrode and the skin.

[Description of Drawings]

FIG. 1 is a diagram for illustrating a basic principle on which a potential difference between measurement electrodes spaced apart from each other is calculated in an ECG measurement model.

FIG. 2 is a block diagram of a defibrillator in accordance with an embodiment of the present invention.

FIG. 3 is a circuit diagram illustrating that the first measurement unit, the second measurement unit, the third measurement unit, and the estimation unit of the defibrillator in accordance with an embodiment of the present invention have been illustratively implemented.

FIG. 4 is a circuit diagram illustrating an exemplary implementation of the signal restoration unit of the defibrillator in accordance with an embodiment of the present invention.

FIG. 5 is a block diagram illustrating a defibrillator extended in accordance with an embodiment of the present invention.

FIG. 6 is a diagram illustrating the configuration of the electrodes of a defibrillator capable of measuring a motion artifact.

FIG. 7 is a flowchart illustrating a method of operating the defibrillator in accordance with an embodiment of the present invention.

[Description of numerals]

200:    defibrillator
210:    pair of first electrodes
220:    pair of second electrodes
230:    first measurement unit
240:    second measurement unit
250:    third measurement unit
260:    estimation unit
270:    signal restoration unit
280:    energy conversion unit
290:    power storage unit
410:    normalization unit
420:    analysis unit
430:    delay unit
440:    FIR filter unit
450:    adaptive filter unit
460:    calculation unit

[Mode for Invention]

Hereinafter, preferred embodiments of the present invention which may realize the objects concretely are described with reference to the accompanying drawings. In this case, the elements and operations of the present invention illustrated in the drawings and described with reference to the drawings are described as at least one embodiment, and the technical idea of the present invention and core configurations and operations thereof are not limited by such elements and operations.

Terms used in the present invention are common terms that are now widely used by taking functions in the present invention into consideration, but the terms may be changed depending on an intention of a person skilled in the art, a use practice, or the appearance of a new technology. Furthermore, in special cases, terms randomly selected by the applicant are used. In such a case, the meaning of a corresponding term is clearly described in a corresponding part of the detailed description. Accordingly, terms used in the present invention should not be understood simply based on their names, but should be understood based on their meanings and the general contents of the present invention.

In the entire specification, an ECG signal is a signal directly obtained from a body through a plurality of electrodes. The ECG signal refers to a signal including the external force induced electrical excitation of the heart due to chest compression, an artifact generated on a boundary surface between an electrode and the skin.

Furthermore, in the entire specification, an ECG artifact is a result obtained by measuring a change in the potential of each electrode attributable to a motion and may be understood as the same meaning as a motion artifact used in the entire specification.

In the entire specification, a low ECG signal is a difference between measured potentials between main electrodes and refers to an ECG signal before an ECG artifact is removed.

Furthermore, in the entire specification, the final ECG is a signal obtained by removing the ECG artifact from the low ECG signal and means a signal to be finally obtained by a defibrillator according to the present invention.

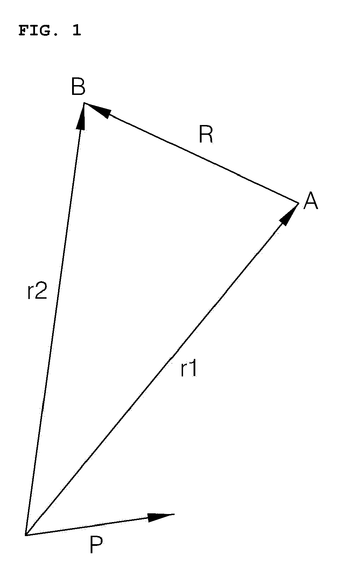

FIG. 1 is a diagram for illustrating a basic principle on which a potential difference between measurement electrodes spaced apart from each other is calculated in an ECG measurement model.

In the ECG measurement model, the heart is considered to be a single electrical dipole, and a potential difference between points A and B in respective distances $r_1$ and $r_2$ in the dipole is computed.

A potential difference between the spaced measurement electrodes is the same as Equation 1. The size of a heart signal is reduced as a distance vector R between electrodes and a distance r between the heart and a measurement electrode are reduced.

$$\text{Equation 1}$$

$$\nu(r_1, r_2) = \frac{\overline{P} \cdot (r_2 - r_1)}{4\pi\sigma_\Phi r^3} = \frac{\overline{P} \cdot \overline{R}}{4\pi\sigma_\Phi r^3}, \overline{R} = r_2 - r_1$$

If the distance from the heart and the distance from the electrode are reduced based on such a principle, only a motion artifact can be emphasized because a Signal to Noise Ratio (SNR) is lowered.

$$\text{Equation 2}$$

$$\text{SNR}_{dB} = 10\log_{10}\left(\frac{P_{signal}}{P_{noise}}\right) = P_{signal,dB} - P_{noise,dB}$$

In other words, a defibrillator according to the present invention identifies a motion artifact using a motion artifact emphasizing method.

If the principle is applied to the present invention, A and B of FIG. 1 may be considered to be a pair of first electrodes and a pair of second electrodes, respectively, in the distances $r_1$ and $r_2$ from the heart. Accordingly, a motion artifact can be identified using an SNR emphasizing method, such as Equation 2.

Furthermore, the principle may also be applied to a main electrode and sub-electrode included in each electrode pair.

Assuming that A' and B' are placed at respective adjacent points in A and B of FIG. 1, A and B may be considered to be a first main electrode and a second main electrode, respectively, and A' and B' may be considered to be a first sub-electrode and a second sub-electrode, respectively. Furthermore, assuming that distances between the heart and A and A' are $r_1$ and $r_1'$, respectively, and distances between the heart and B and B' are $r_2$ and $r_2'$, respectively, a motion artifact occurred in each electrode can be identified using the SNR emphasizing method of Equation 2.

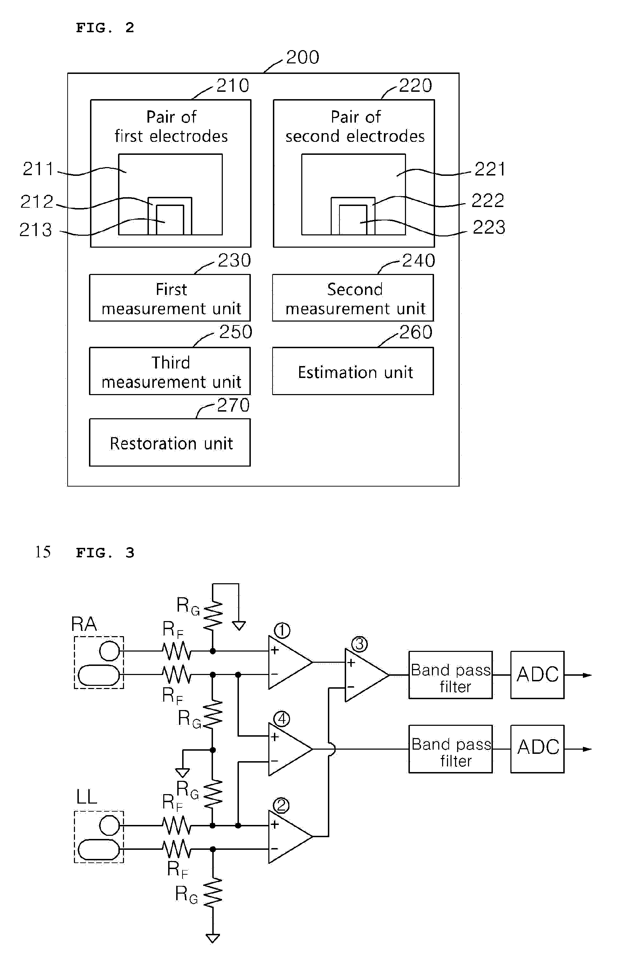

Contents regarding the measurement of a motion artifact between the pair of first electrodes and the pair of second electrodes and between the main electrode and sub-electrode included in each of the electrode pairs are described in detail later with reference to FIG. 3.

FIG. 2 is a block diagram of a defibrillator 200 in accordance with an embodiment of the present invention.

The defibrillator 200 is configured to include a pair of first electrodes 210, a pair of second electrodes 220, a first measurement unit 230, a second measurement unit 240, a third measurement unit 250, an estimation unit 260, and a signal restoration unit 270.

The pair of first electrodes 210 includes a first main electrode 211 and a first sub-electrode 213 placed adjacent to the first main electrode 211 with an insulation unit 212 interposed between the first main electrode and the first sub-electrode 213. Likewise, the pair of second electrodes 220 includes a second main electrode 221 and a second sub-electrode 223 placed adjacent to the second main electrode and insulated from the second main electrode 221 through an insulation unit 222. The ECG signal of an object is received through the main electrodes and sub-electrodes of the pair of first electrodes 210 and the pair of second electrodes 220.

The first measurement unit 230 measures a first active potential, associated with the pair of first electrodes 210, using a potential difference between the first main electrode 211 and first sub-electrode 213 of the pair of first electrodes 210.

Likewise, the second measurement unit 240 measures a second active potential, associated with the pair of second electrodes 220, using a potential difference between the second main electrode 221 and second sub-electrode 223 of the pair of second electrodes 220.

The estimation unit 260 performs the estimation of an ECG artifact associated with a difference between the first active potential and the second active potential. The ECG artifact corresponds to a noise that is representative of a change of an electrical characteristic within an object attributable to external physical force that is transferred to at least one of the plurality of electrodes of the defibrillator 200 of the present invention or the object.

The estimation unit 260 is configured to include a first amplifier for amplifying a difference between the first active potential and the second active potential respectively measured by the first measurement unit 230 and the second measurement unit 240, a first bandpass filter unit for performing a bandpass operation on the output of the first amplifier in response to a first frequency band, and a first analog-digital converter unit for converting the bandpass-filtered signal into a digital signal.

The third measurement unit 250 measures a low ECG signal using a difference between the potentials of the first main electrode 211 and the second main electrode 221.

The third measurement unit 250 is configured to include a second amplifier for amplifying a difference between the potential of the first main electrode 211 and the potential of the second main electrode 221, a second bandpass filter unit for performing a bandpass operation on the output of the second amplifier in response to a second frequency band, and a second analog-digital converter unit for converting the bandpass-filtered signal into a digital signal.

The estimation unit 260 restores the final ECG obtained by removing an ECG artifact from the low ECG signal using the ECG artifact estimated by the estimation unit 260.

The signal restoration unit 270 may be configured to include an adaptive filter unit for filtering the ECG artifact estimated by the estimation unit 260, a delay unit for delaying the low ECG signal measured by the third measurement unit, and a calculation unit for restoring the final ECG signal by subtracting the filtered ECG artifact from the delayed low ECG signal.

FIG. 3 is a circuit diagram illustrating that the first measurement unit 230, the second measurement unit 240, the third measurement unit 250, and the estimation unit 260 of the defibrillator in accordance with an embodiment of the present invention have been illustratively implemented.

Potentials measured from the pair of first electrodes and the pair of second electrodes may be expressed as in Equation 3.

Equation 3

$$X = Heart_{RM} + e_{RM} \qquad X' = Heart_{RS} + e_{RS}$$

$$Y = Heart_{LM} + e_{LM} \qquad Y' = Heart_{LS} + e_{LS}$$

In Equation 3, $Heart_{xx}$ is a heart potential measured from the pair of first electrodes (the first main electrode and the first sub-electrode) and the pair of second electrodes (the second main electrode and the second sub-electrode), and $e_{xx}$ is the active potential of each of the electrodes. $X$ and $X'$ denote changes of potentials measured from the first main electrode and the first sub-electrode, and $Y$ and $Y'$ denote changes of potentials measured from the second main electrode and the second sub-electrode.

A change of electric potential between the first main electrode 211 and the second main electrode 221 that is measured by the third measurement unit 250 is the same as Equation 4.

Equation 4

$$X-Y = (Heart_{RM}+e_{RM})-(Heart_{LM}+e_{LM})$$

$$= \Delta Heart_M + \Delta e_M$$

In Equation 4, $\Delta Heart_M$ means a common ECG, and $\Delta e_M$ means a difference between the active potentials of the main electrodes of the pair of first electrodes and the pair of second electrodes.

A potential difference between the first main electrode 211 and the first sub-electrode 213, measured by the first measurement unit 230, and a potential difference between the second main electrode 221 and the second sub-electrode 223, measured by the second measurement unit 240, is the same as Equation 5.

Equation 5

$$N_R = X-X' = (Heart_{RM}+e_{RM})-(Heart_{RS}+e_{RS})$$

$$= (Heart_{RM}+Heart_{RS})-(e_{RM}+e_{RS})$$

$$= \Delta Heart_R + \Delta e_R$$

$$N_L = Y-Y' = (Heart_{LM}+e_{LM})-(Heart_{LS}+e_{LS})$$

$$= (Heart_{LM}+Heart_{LS})-(e_{LM}+e_{LS})$$

$$= \Delta Heart_L + \Delta e_L$$

In Equation 5, $N_R$ and $N_L$ mean respective potential differences between the main electrodes 211 and 221 and the respective sub-electrodes 213 and 223, measured by the first measurement unit 230 and the second measurement unit 240, that is, the first active potential and the second active potential.

A difference between the first active potential and the second active potential measured by the estimation unit 260 may be expressed by Equation 6.

Equation 6

$$N_{RL} = N_R - N_L = (\Delta Heart_R + \Delta e_R)-(\Delta Heart_L + \Delta e_L)$$

$$= (\Delta Heart_R + \Delta Heart_L)+(\Delta e_R + \Delta e_L)$$

In this case, assuming that $\Delta Heart_R$ and $\Delta Heart_L$ are neglected because they have relatively small values, the result of Equation 6 may be considered to be $N_{RL} \approx \Delta e_R - \Delta e_L$. Furthermore, the result $N_{RL}$ of Equation 6 is estimated as an ECG artifact.

Furthermore, if the area of the electrode has a relationship proportional to the influence of an active potential, the active potentials $\Delta e_{RM}$ and $\Delta e_{LM}$ of the first main electrode and the second main electrode may be estimated as $\Delta e_R$ and $\Delta e_L$, respectively. That is, it may be considered to be $\Delta e_M \approx N_{RL}$.

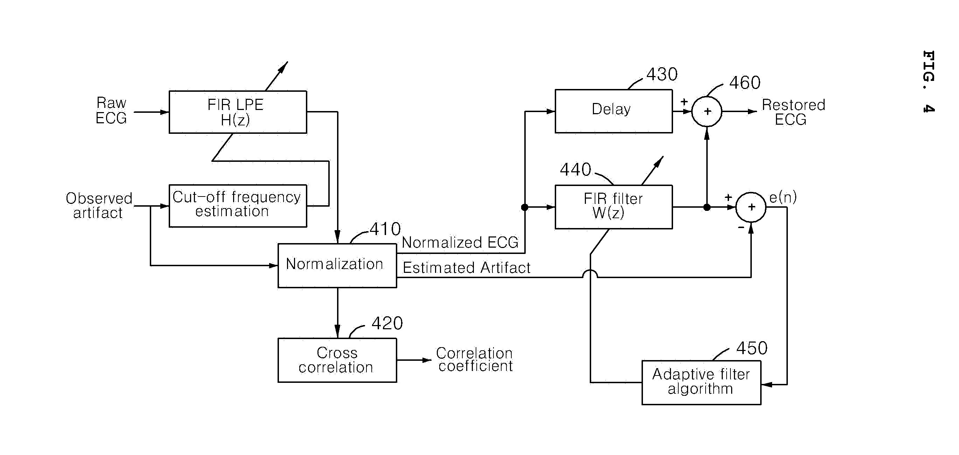

FIG. 4 is a circuit diagram illustrating an exemplary implementation of the signal restoration unit 270 of the defibrillator in accordance with an embodiment of the present invention.

The signal restoration unit 270 is configured to include a normalization unit 410 for normalizing an ECG artifact estimated by the estimation unit 260 and a low ECG signal measured by the third measurement unit 250, an analysis unit 420 for determining a cross correlation between the normalized signals, a delay unit 430 for delaying and filtering the normalized ECG signal, an FIR filter unit 440, an adaptive filter unit 450 for restoring the normalized ECG signal and the ECG artifact, and a calculation unit 460 for restoring the final ECG signal by subtracting the filtered ECG artifact from the delayed low ECG signal.

Referring to FIG. 4, the signal restoration unit 270 analyzes a frequency characteristic of an ECG artifact received from the estimation unit and sets a low bandpass band so that the band is time-variant. Furthermore, the signal restoration unit 270 extracts a base line component similar to a change of the ECG artifact from a low ECG signal that has been measured by the third measurement unit and received and scales the base line component by normalizing the base line component through the normalization unit 410.

The cross correlation between the normalized signals is read through a cross correlation analysis performed by the analysis unit 420. The reading of the cross correlation is performed using a correlation coefficient obtained as a result of the cross correlation analysis. If the correlation coefficient has a value smaller than a specific value, the ECG artifact signal is determined to be unreliable.

For example, assuming that the correlation coefficient becomes numerical in a range of 0~1 and a specific reference is 0.7, if the correlation coefficient is smaller than 0.7, measurement needs to be performed again because the ECG artifact signal is unreliable.

However, if the correlation coefficient is equal to or greater than 0.7, the normalized ECG signal and the normalized ECG artifact signal may be obtained because the ECG artifact signal is reliable.

The normalized ECG signal and the normalized ECG artifact are restored by the adaptive filter unit 450.

The normalized ECG signal is subject to the operations of the delay unit 430 and the FIR filter unit 440 and is subject to subtraction operation along with the ECG artifact filtered by the calculation unit 460, thus being restored to the final ECG signal (i.e., a restored ECG).

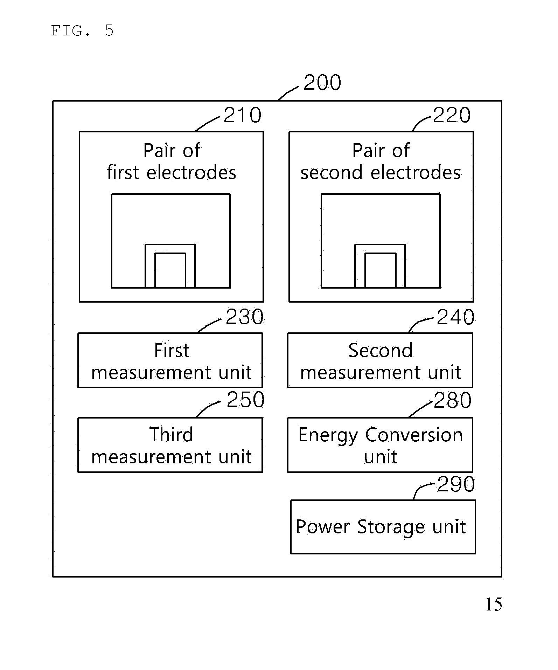

FIG. 5 is a block diagram illustrating a defibrillator 200 extended in accordance with an embodiment of the present invention.

The defibrillator 200 is configured to include a pair of first electrodes 210, a pair of second electrodes 220, a first measurement unit 230, a second measurement unit 240, a third measurement unit 250, an estimation unit 260, an energy conversion unit 280, and a power storage unit 290.

The pair of first electrodes 210, the pair of second electrodes 220, the first measurement unit 230, the second measurement unit 240, the third measurement unit 250, and the estimation unit 260 are the same as those illustrated in FIG. 2. The defibrillator 200 may be configured to further include the energy conversion unit 280 and the power storage unit 290 in addition to the elements of FIG. 2.

The energy conversion unit 280 receives an electromagnetic signal around the defibrillator 200 and converts the electromagnetic signal into electric energy.

The energy conversion unit 280 includes a power monitoring unit for collecting information about a power supply state of the defibrillator 200 and a controller for transferring the abnormality of power supply to an external device if the abnormality of power supply is detected based on the information about the power supply state received from the power monitoring unit. The energy conversion unit 280 may further include a communication processor responsible for communication between the defibrillator and the external device and a diagnosis processor for checking the current state of power supply based on information about a power supply state collected by the power monitoring unit.

The controller automatically instructs internal power supply if power supply through the energy conversion unit 280 is impossible. Furthermore, the controller checks a power supply problem through the power monitoring unit. If the amount of remaining battery power is less than or equal to the minimum amount for defibrillation, the controller transfers a communication function stop message to an external device and blocks the function of a communication process.

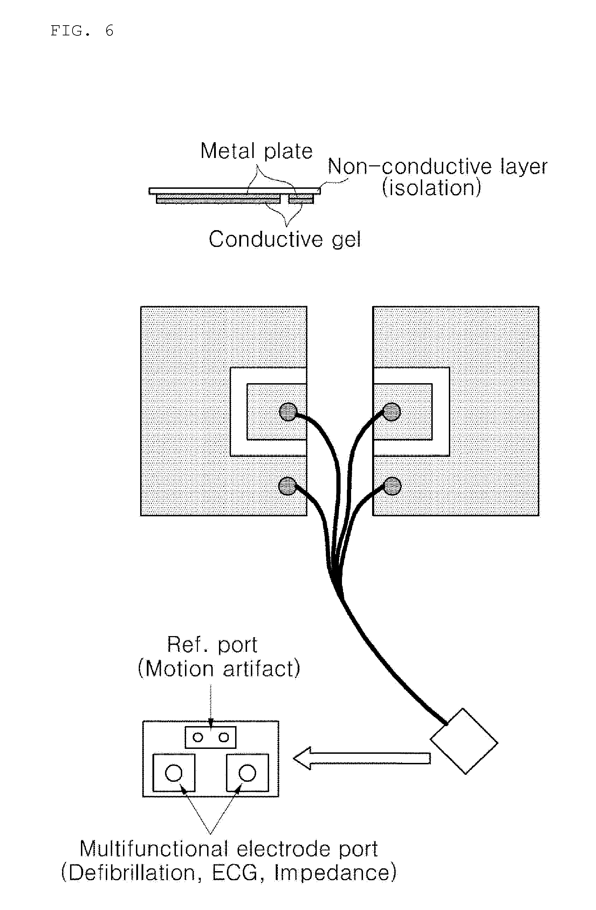

FIG. 6 is a diagram illustrating the configuration of the electrodes of the defibrillator capable of measuring a motion artifact.

In order to simultaneously measure an ECG and a motion artifact, electrode pairs having a structure, such as that of FIG. 6, are used. Each of the electrode pairs includes two separated conduction parts with an insulation unit interposed in between. The two separated conduction parts may be considered to be a main electrode and a sub-electrode, respectively. The main electrode and the sub-electrode are connected to connectors through separated lines.

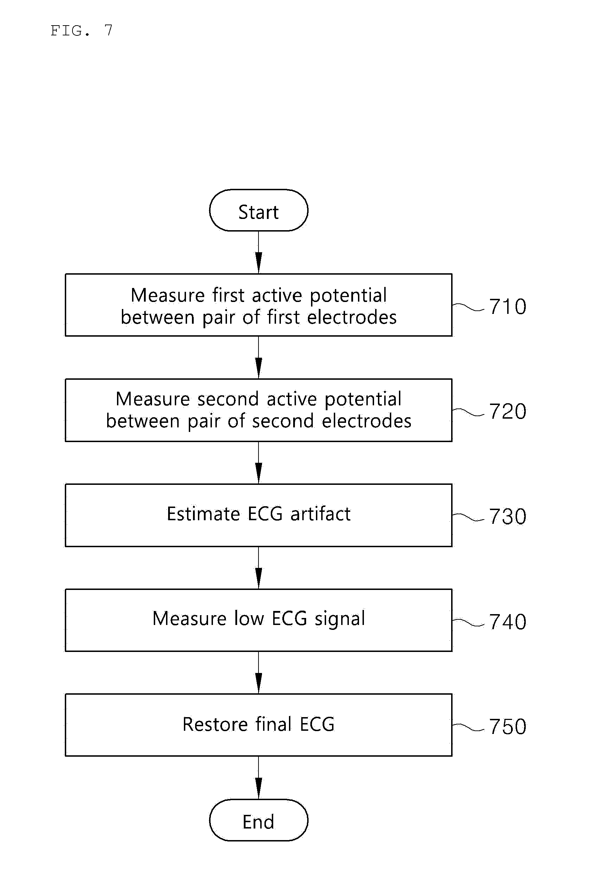

FIG. 7 is a flowchart illustrating a method of operating the defibrillator for monitoring the ECG signal of an object using a plurality of electrodes in accordance with an embodiment of the present invention.

At step 710, a first active potential associated with the pair of first electrodes is measured using a potential difference between the first main electrode 211 and first sub-electrode 213 of the pair of first electrodes 210.

The first main electrode 211 and first sub-electrode 213 of the pair of first electrodes 210 are placed adjacent to each other and insulated from each other with the insulation unit 212 interposed therebetween.

At step 720, a second active potential associated with the pair of second electrodes is measured using a potential difference between the second main electrode 221 and second sub-electrode 223 of the pair of second electrodes 220.

Like the pair of first electrodes 210, the pair of second electrodes 220 is configured to include the second main electrode 221 and the second sub-electrode 223 that is insulated from and placed adjacent to the second main electrode.

The first active potential and the second active potential measured at steps 710 and 720 are used to estimate an ECG artifact at step 730. The ECG artifact is estimated in association with a difference between the first active potential and the second active potential.

As described with reference to FIG. 3, the estimated ECG artifact is obtained in such a way as to amplify a difference between the first active potential and the second active potential, perform a bandpass operation on the amplified output in response to a first frequency band, and convert the bandpass-filtered signal into a digital signal.

The ECG artifact corresponds to a noise that is representative of a change of an electrical characteristic within an object attributable to external physical force that is transferred to at least one of the plurality of electrodes of the defibrillator of the present invention or the object.

At step 740, a low ECG signal is measured using a potential difference between the first main electrode 211 and the second main electrode 221.

The low ECG signal may be obtained in such a way as to amplify a difference between the potential of the first main electrode and the potential of the second main electrode, perform a bandpass operation on the amplified output in response to a second frequency band, and convert the bandpass-filtered signal into a digital signal.

At step 750, the final ECG obtained by removing an artifact from the low ECG signal is restored using the estimated ECG artifact.

The final ECG is restored by delaying the low ECG signal, filtering the ECG artifact, and then subtracting the filtered ECG artifact from the delayed low ECG signal.

As described above, although the present invention has been described in connection with specific matters, such the detailed elements, and the limited embodiments and drawings, they are provided only to help general understanding of the present invention, and the present invention is not limited to the embodiments. A person having ordinary skill in the art to which the present invention pertains may change and modify the present invention in various ways from the above description.

Accordingly, the spirit of the present invention should not be construed as being limited to the embodiments, and all the equal or equivalent modifications of the attached claims should be constructed as belonging to the category of the present invention.

## Claims

1. A defibrillator for monitoring an ECG signal of an object using a plurality of electrodes, comprising:

   a pair of first electrodes comprising a first main electrode and a first sub-electrode placed adjacent to the first main electrode and insulated from the first main electrode;
   a pair of second electrodes comprising a second main electrode and a second sub-electrode placed adjacent to the second main electrode and insulated from the second main electrode;
   a first measurement unit measuring a first active potential, associated with the pair of first electrodes, using a potential difference between the pair of first electrodes;
   a second measurement unit measuring a second active potential, associated with the pair of second electrodes, using a potential difference between the pair of second electrodes; and
   an estimation unit estimating an ECG artifact associated with a difference between the first active potential and the second active potential.

2. The defibrillator of claim 1, further comprising:

   a third measurement unit measuring a low ECG signal using a potential difference between the first main electrode and the second main electrode; and
   a signal restoration unit restoring a final ECG obtained by removing the ECG artifact from the low ECG signal using the estimated ECG artifact.

3. The defibrillator of claim 2, wherein the signal restoration unit comprises:

   an adaptive filter unit filtering the ECG artifact;
   a delay unit delaying the low ECG signal; and
   a calculation unit restoring the final ECG signal by subtracting the filtered ECG artifact from the delayed low ECG.

4. The defibrillator of claim 1, wherein the estimation unit comprises:

   a first amplifier amplifying the difference between the first active potential and the second active potential;
   a first bandpass filter unit performing a bandpass operation on an output of the first amplifier in response to a first frequency band; and
   a first analog-digital converter unit converting the bandpass-filtered signal into a digital signal.

5. The defibrillator of claim 2, wherein the third measurement unit comprises:

   a second amplifier amplifying a difference between the potential of the first main electrode and the potential of the second main electrode;
   a second bandpass filter unit performing a bandpass operation on an output of the second amplifier in response to a second frequency band; and
   a second analog-digital converter unit converting the bandpass-filtered signal into a digital signal.

6. The defibrillator of claim 1, wherein the ECG artifact corresponds to a noise representative of a change of an electrical characteristic within an object attributable to external physical force that is transferred to at least one of the plurality of electrodes of the defibrillator or the object.

7. The defibrillator of claim 1, further comprising:

   an energy conversion unit receiving an electromagnetic signal around the defibrillator and converting the electromagnetic signal into electric energy; and
   a power storage unit storing the electric energy converted by the energy conversion unit and supplying driving power for the defibrillator.

8. The defibrillator of claim 7, wherein the energy conversion unit comprises:

   a power monitoring unit collecting information about a power supply state of the defibrillator; and
   a controller transferring an abnormality of power supply to an external device if the abnormality of power supply is detected based on the information about the power supply state received from the power monitoring unit.

9. The defibrillator of claim 8, wherein the controller automatically instructs internal power supply if power supply through the energy conversion unit is impossible.

10. An electrode unit of a defibrillator brought in contact with an object in order to measure an ECG signal of the object, the electrode unit comprising:

    a pair of first electrodes comprising a first main electrode and a first sub-electrode placed adjacent to the first main electrode and insulated from the first main electrode; and
    a pair of second electrodes comprising a second main electrode and a second sub-electrode placed adjacent to the second main electrode and insulated from the second main electrode.

11. A method of operating a defibrillator for monitoring an ECG signal of an object using a plurality of electrodes, the method comprising steps of:

    measuring a first active potential associated with a pair of first electrodes using a potential difference between the pair of first electrodes of the defibrillator - the pair of first electrodes comprising a first main electrode and a first sub-electrode placed adjacent to the first main electrode and insulated from the first main electrode -;
    measuring a second active potential associated with a pair of second electrodes using a potential difference between the pair of second electrodes of the defibrillator - the pair of second electrodes comprising a second main electrode and a second sub-electrode placed adjacent to the second main electrode and insulated from the second main electrode -; and
    estimating an ECG artifact associated with a difference between the first active potential and the second active potential.

12. The method of claim 11, further comprising steps of:

    measuring a low ECG signal using a potential difference between the first main electrode and the second main electrode; and
    restoring a final ECG obtained by removing an artifact from the low ECG signal using the estimated ECG artifact.

FIG. 1

FIG. 2

FIG. 3

FIG. 4

FIG. 5

200

210
Pair of
first electrodes

220
Pair of
second electrodes

230
First
measurement unit

240
Second
measurement unit

250
Third
measurement unit

280
Energy Conversion
unit

290
Power Storage unit

15

FIG. 6

Metal plate

Non-conductive layer
(isolation)

Conductive gel

Ref. port
(Motion artifact)

Multifunctional electrode port
(Defibrillation, ECG, Impedance)

FIG. 7

```
                    ╭─────────╮
                    │  Start  │
                    ╰─────────╯
                         │
                         ▼
        ┌───────────────────────────────────┐
        │      Measure first active potential │ ⌐~ 710
        │    between pair of first electrodes  │
        └───────────────────────────────────┘
                         │
                         ▼
        ┌───────────────────────────────────┐
        │     Measure second active potential │ ⌐~ 720
        │   between pair of second electrodes  │
        └───────────────────────────────────┘
                         │
                         ▼
        ┌───────────────────────────────────┐
        │          Estimate ECG artifact      │ ⌐~ 730
        └───────────────────────────────────┘
                         │
                         ▼
        ┌───────────────────────────────────┐
        │          Measure low ECG signal     │ ⌐~ 740
        └───────────────────────────────────┘
                         │
                         ▼
        ┌───────────────────────────────────┐
        │           Restore final ECG         │ ⌐~ 750
        └───────────────────────────────────┘
                         │
                         ▼
                    ╭─────────╮
                    │   End   │
                    ╰─────────╯
```

## INTERNATIONAL SEARCH REPORT

International application No.

**PCT/KR2013/003618**

### A. CLASSIFICATION OF SUBJECT MATTER

***A61N 1/39(2006.01)i, A61N 1/04(2006.01)i, A61B 5/0408(2006.01)i***

According to International Patent Classification (IPC) or to both national classification and IPC

### B. FIELDS SEARCHED

Minimum documentation searched (classification system followed by classification symbols)
A61N 1/39; A61H 31/00; A61N 1/362; A61B 5/02; A61B 5/0205; A61B 5/0402; A61M 16/00; A61B 5/0408; A61N 1/04

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: defibrillator, ECG, noise, removal, electrode, amplification, filtering

### C. DOCUMENTS CONSIDERED TO BE RELEVANT

| Category* | Citation of document, with indication, where appropriate, of the relevant passages | Relevant to claim No. |
|---|---|---|
| X | KR 10-2011-0135296 A (SAMSUNG ELECTRONICS CO., LTD.) 16 December 2011 See abstract, paragraphs [0017]-[0022], [0048]-[0049], claims 1-4 and figures 1, 3, 4 and 13. | 1-6,10-12 |
| A | | 7-9 |
| A | KR 10-2008-0097934 A (BIOSENSE WEBSTER, INC.) 06 November 2008 See abstract, paragraphs [0027]-[0032], claims 1, 7 and 10 and figure 2. | 1-12 |
| A | JP 2011-161258 A (ZOLL CIRCULATION INC) 25 August 2011 See abstract, paragraphs [0020]-[0026], claim 1 and figure 1. | 1-12 |
| A | JP 2004-249080 A (HARBINGER MEDICAL INC) 09 September 2004 See abstract, paragraphs [0019]-[0024], claim 1 and figures 1-4. | 1-12 |
| A | KR 10-2002-0021621 A (CHOI, Sung Hwan et al.) 21 March 2002 See abstract, claim 1 and figure 3. | 1-12 |

☐ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 JULY 2013 (29.07.2013) | **29 JULY 2013 (29.07.2013)** |

| Name and mailing address of the ISA/KR | Authorized officer |
|---|---|
| Korean Intellectual Property Office Government Complex-Daejeon, 189 Seonsa-ro, Daejeon 302-701, Republic of Korea | |
| Facsimile No. 82-42-472-7140 | Telephone No. |

Form PCT/ISA/210 (second sheet) (July 2009)

INTERNATIONAL SEARCH REPORT
Information on patent family members

International application No.

**PCT/KR2013/003618**

| Patent document cited in search report | Publication date | Patent family member | Publication date |
|---|---|---|---|
| KR 10-2011-0135296 A | 16/12/2011 | CN 102283641 A | 21/12/2011 |
| | | EP 2394571 A1 | 14/12/2011 |
| | | JP 2011-255187A | 22/12/2011 |
| | | US 2011-0306892 A1 | 15/12/2011 |
| KR 10-2008-0097934 A | 06/11/2008 | AU 2008-201890 A1 | 20/11/2008 |
| | | CA 2630019 A1 | 02/11/2008 |
| | | CN 101297754 B | 08/08/2012 |
| | | EP 1987767 B1 | 11/08/2010 |
| | | JP 05-191791B2 | 08/05/2013 |
| | | JP 2008-284352A | 27/11/2008 |
| | | US 2008-0275353 A1 | 06/11/2008 |
| | | US 7894885 B2 | 22/02/2011 |
| JP 2011-161258 A | 25/08/2011 | AU 2003-291656 A1 | 13/05/2004 |
| | | AU 2009-238264 B2 | 20/12/2012 |
| | | CA 2503544 C | 31/07/2012 |
| | | CA 2776907 A1 | 06/05/2004 |
| | | EP 1558193 A2 | 03/08/2005 |
| | | EP 2532338 A2 | 12/12/2012 |
| | | EP 2532339 A2 | 12/12/2012 |
| | | EP 2532340 A2 | 12/12/2012 |
| | | JP 04-762545B2 | 31/08/2011 |
| | | JP 05-165079B2 | 21/03/2013 |
| | | JP 2006-503659A | 02/02/2006 |
| | | JP 2010-214122A | 30/09/2010 |
| | | JP 2012-166082A | 06/09/2012 |
| | | US 2004-0082888 A1 | 29/04/2004 |
| | | US 2004-0210170 A1 | 21/10/2004 |
| | | US 2004-0210171 A1 | 21/10/2004 |
| | | US 2004-0210172 A1 | 21/10/2004 |
| | | US 2007-0010764 A1 | 11/01/2007 |
| | | US 2009-112135 A1 | 30/04/2009 |
| | | US 2013-060172 A1 | 07/03/2013 |
| | | US 2013-060173 A1 | 07/03/2013 |
| | | US 6827695 B2 | 07/12/2004 |
| | | US 7118542 B2 | 10/10/2006 |
| | | US 7122014 B2 | 17/10/2006 |
| | | US 7476206 B2 | 13/01/2009 |
| | | US 8096962 B2 | 17/01/2012 |
| | | WO 2004-037154 A2 | 06/05/2004 |
| JP 2004-249080 A | 09/09/2004 | DE 60313830 T2 | 31/01/2008 |
| | | EP 1384434 B1 | 16/05/2007 |
| | | US 2004-0054383 A1 | 18/03/2004 |
| | | US 7016731 B2 | 21/03/2006 |
| KR 10-2002-0021621 A | 21/03/2002 | NONE | |